# EUROPEAN PATENT SPECIFICATION

(11) **EP 4 277 579 B1**
(45) Date of publication and mention of the grant of the patent: **08.10.2025**
(21) Application number: 22700150.0
(22) Date of filing: 06.01.2022
(51) Int. Cl.: A61F 2/32, A61F 2/34, A61F 2/36, A61F 2/38, A61F 2/44, A61F 2/30

(54) **ORTHOPAEDIC IMPLANT**
ORTHOPÄDISCHES IMPLANTAT
IMPLANT ORTHOPÉDIQUE

(30) Priority: 15.01.2021 GB 202100575
(43) Date of publication of application: 22.11.2023
(73) Proprietor: Osstec Limited, London SW7 1BU (GB)
(72) Inventor: MUNFORD, Maxwell, London SW7 1BU (GB)
(74) Representative: HGF
(86) International application number: PCT/GB2022/050016
(87) International publication number: WO 2022/153033

(56) References cited:
- EP-A1- 3 388 029
- CN-A- 108 742 952
- CN-A- 111 449 808
- CN-A- 112 155 796
- US-A1- 2018 296 350

## Description

This invention is defined in claim 1 and relates generally to an orthopaedic implant, such as a modular knee, hip or spinal implant.

### BACKGROUND

Conventional orthopaedic implants, such as tibial, femoral and acetabular components, and spinal fusion cages, are known. These implants are typically cast as a solid single piece component, and are manufactured from titanium. One problem with solid titanium implants is that titanium has an elastic modulus which is greater than that of the bone into which the implant will be implanted. This can lead to stress shielding of the surrounding bone which can result in bone loss, implant loosening and the need for revision surgery. A further disadvantage of solid titanium implants is the considerably increased load distribution across the articulating surface which is not representative of the load distribution in healthy joints where loads are typically focussed under the contact points of neighbouring articulating surfaces, such as under the femoral condyles.

Conventional orthopaedic implants of this kind are often cemented or glued into the bone material, or are attached with pins or other fixings, or are simply secured by impacting the implant into the bone to achieve an adequate level of mechanical fixation.

Conventional solid titanium implants provide very little biomechanical stimulation of the surrounding bone material and typically install strain in the order of 0.01 microstrain (µm*m⁻¹) in the bone material surrounding the implant.

Whilst the specific levels of strain necessary to induce particular physiological responses will vary between people, it is broadly understood that:
- strain rates of less than approximately 50 microstrain in bone material results in a reduction in bone mass;
- strain between around 50 µm*m⁻¹ microstrain and 200 µm*m⁻¹ microstrain results in bone remodelling;
- strain between around 2000 µm*m⁻¹ microstrain and 3000 µm*m⁻¹ microstrain results in bone modelling; and
- strain of around 4000 µm*m⁻¹ microstrain results in bone repair.

Under loading conditions of 0.5 to 2 times the bodyweight of the patient, loads representative of most activities of daily living, such as walking, standing, getting up and sitting down, the conventional implant will undergo negligible strain, which also results in negligible strain in the surrounding bone. This results in bone loss, or resorption, and implant loosening over time as the levels of strain installed into the bone are considerably below those required to achieve bone remodelling or modelling. This also leads to premature aging, for example cracking, of the bone material in the vicinity of the implant.

It is known to use foam materials, for example tantalum foam, in implants. The foam material provides a porous surface for bone in-growth into the implant. However, the pores of the foam material are evenly distributed, and so the strain in the surrounding bone is inconsistent, as the elastic modulus of bone varies across its length. Furthermore, the range of elastic modulus that can be achieved by the foam material is limited by the ability to distribute the pores whilst maintaining structural integrity of the foam. CN111449808 discloses an additive-manufactured porous tantalum metal acetabular outer cup and a preparation method thereof. US2018/296350 describes an implant which includes a body including a substrate and a bone interfacing lattice disposed on the substrate. The bone interfacing lattice includes at least two layers of elongate curved structural members. In addition, the at least two layers of elongate curved structural members include a first layer adjacent the substrate and a second layer adjacent the first layer. Also, the first layer has a first deformability and the second layer has a second deformability, wherein the second deformability is greater than the first deformability.

The present invention seeks to address at least some of these problems.

### BRIEF SUMMARY OF THE DISCLOSURE

According to a first embodiment, there is provided an orthopaedic implant according to claim 1.

This is advantageous as, under normal loading conditions in activities of daily living the implant itself will deform by much larger rates than previously seen, which will in turn install sufficiently high levels of strain in the bone contacting the engaging surface of the implant in a wide range of patients, whose bone quality will range from very stiff to very compliant, to avoid the deleterious effects of solid titanium implants. This increased strain, compare to solid titanium implants, greatly reduces, or even removes, bone loss in the majority of patients and enables the bone to grow into the lattice structure to provide adequate fixation of the implant. Over the lifetime of the implant, the deformable lattice structure will also induce greater levels of strain in the surrounding bone which will lead to remodelling of the bone material surrounding the implant in a natural way. A further advantage of the present implant is that a lattice structure enables the elastic modulus to be tuned in a specific manner, for example to have a non-uniform elastic modulus across the implant surface, such that the range of strain installed in the bone can also be tuned as desired in order to achieve good levels of bone in-growth into the lattice. This provides greater ability to fine-tune the mechanical properties of the implant compared to porous implants.

There is also provided an orthopaedic implant comprising a securing element configured to engage an external component; and a lattice structure connected to the securing element and having an engaging surface configured, in use, to engage a bone surface. The securing element provides a load path between the external component and the lattice structure. The lattice structure has an elastic modulus of between 0.1 Gpa and 20 Gpa, such that the engaging surface is configured to deform by between 5 µm.m⁻¹ (microstrain) and 6000 µm.m⁻¹ (microstrain) upon application of a load by the external component. The lattice structure comprises a second surface which is arranged, in use, to abut the articulating joint component.

A further advantage of the lattice structure is that the elastic modulus can be tuned depending on the joint the implant is to be implanted into, or the quality of bone it is to be implanted into, and so the lattice structure does not need to be designed from scratch to account for patient specific requirements.

The lattice structure may comprise a second surface which is arranged, in use, to abut the external component. The second surface may be on an opposed side of the lattice structure relative to the engaging surface.

The engaging surface may be configured to deform by between 30 µm*m⁻¹ (microstrain) and 6000 µm*m⁻¹ (microstrain) upon application of a load by the external component. The engaging surface may be configured to deform by between 50 µm*m⁻¹ (microstrain) and 4000 µm*m⁻¹ (microstrain) upon application of a load by the external component. The engaging surface may be configured to deform by between 50 µm*m⁻¹ (microstrain) and 200 µm*m⁻¹ (microstrain) upon application of a load by the external component. The engaging surface may be configured to deform by between 2000 µm*m⁻¹ (microstrain) 3000 µm*m⁻¹ (microstrain) upon application of a load by the external component. The engaging surface may be configured to deform by between 4000 µm*m⁻¹ (microstrain) and 6000 µm*m⁻¹ (microstrain) upon application of a load by the external component.

This is advantageous as the elastic modulus of the bone surrounding the implant varies across the bone surface, and so the elastic modulus of the regions of the implant can be varied such that they correspond to the different elastic modulus of bone surrounding the implant. This means that the strain installed in the bone surrounding the implant can be made more consistent around the entire implant.

Grading the elastic modulus of the intermediate region is advantageous as this more closely approximates the level of strain installed in native bone. Furthermore, by grading the lattice structure, it is possible to provide a lattice structure that is both sufficiently strong and which also has a stiffness at the bone interface that more closely resembles that of native bone to encourage bone in-growth into the lattice structure.

The elastic modulus of the intermediate region may be graded linearly in the first direction. The first direction may correspond to a width or depth of the implant. The first direction may correspond to any of a medio-lateral direction of the implant, an antero-posterior direction of the implant or a superior-inferior direction of the implant. In some cases, the entire lattice structure is graded, for example where the intermediate region makes up a majority of the lattice structure. In some cases the intermediate regions extends from the engaging surface to the second surface. Where the implant is a tibial component, the intermediate region may extend from the second surface to a distal end of the keel.

The width of the intermediate region in the first direction may be less than the width of the first and second regions in the first direction.

A portion of the intermediate region between the first and second regions may have a width of at least 3mm.

The elastic modulus of the lattice structure adjacent the engaging surface may be less than the elastic modulus of a proximal portion of the lattice structure spaced from the engaging surface. This advantageously reduces the stress shielding effects of the implant closer to the bone surface as more of the load will be transferred to the bone in contact with the lattice compared to the lattice structure proximal to the engaging surface. For example, the lattice structure adjacent the surface which abuts the external component may have a greater elastic modulus than the lattice structure adjacent the engaging surface.

The lattice structure may comprise a central portion and a peripheral portion. The elastic modulus of the peripheral portion may be less than the elastic modulus of the central portion.

The securing member may comprise an opening. The second surface of the lattice structure may be arranged within the opening.

The securing member may comprise a band arranged around a portion of the lattice structure. The securing element may be integral with the lattice structure.

The securing element may restrain the external component in a fixed position relative to the orthopaedic implant.

The elastic modulus of the securing member may be greater than the elastic modulus of the lattice structure.

According to a second example, the present disclosure provides an orthopaedic implant comprising a securing element connected to an articulating joint component, and a lattice structure connected to the securing element, and having an engaging surface arranged, in use, to engage a bone surface. The securing element provides a load path between the articulating joint component and the lattice structure. The lattice structure has an elastic modulus of between 0.1 GPa and 20 GPa, such that the engaging surface is configured to deform by between 5 µm*m⁻¹ (microstrain) and 6000 µm*m⁻¹ (microstrain) upon application of a load by the articulating joint component. The lattice structure comprises a second surface which is arranged, in use, to abut the articulating joint component.

This advantageously provides a monobloc implant where the securing component is integral with the lattice structure.

There is also provided an orthopaedic implant comprising a securing element connected to an external component; and a lattice structure connected to the securing element and having an engaging surface configured, in use, to engage a bone surface. The securing element provides a load path between the external component and the lattice structure. The lattice structure has an elastic modulus of between 0.1 GPa and 20 GPa, such that the engaging surface is configured to deform by between 5 µm*m⁻¹ (microstrain) and 6000 µm*m⁻¹ (microstrain) upon application of a load by the external component. The lattice structure comprises a second surface which is arranged, in use, to abut the articulating joint component.

The lattice structure may have a second surface arranged to abut the external component.

The lattice structure may have an elastic modulus between 3 GPa and 20 GPa. In some cases, the bulk modulus of the implant may be the same across the entire implant. In some cases, the different regions of the implant may have different bulk elastic moduli, or the same bulk elastic moduli.

The external component may be an articulating joint component, such as a polymer component.

The orthopaedic implant may be any of a modular knee implant or a modular hip implant. Where the orthopaedic implant is a modular knee implant, the orthopaedic implant may be configured as any of a tibial component or a femoral component. Where the orthopaedic implant is a modular hip implant the orthopaedic implant may be configured as an acetabular cup.

The orthopaedic implant may be configured as any of a tibial component or a femoral component.

According to a third example, the present disclosure provides an orthopaedic implant comprising a lattice structure comprising a first region having a first elastic modulus, and a first surface configured to interface with a first bone surface, and a second surface configured to interface with a second bone surface, a second region having a second elastic modulus different to the first elastic modulus, and an intermediate region between the first and second regions. The elastic modulus of the intermediate region is graded in a first direction extending from the first region to the second region, such that the elastic modulus of the intermediate region varies from the first elastic modulus to the second elastic modulus in the first direction.

Any of the first, second and intermediate regions may have an elastic modulus of between 0.1 GPa and 20 GPa, such that the second surface is configured to deform by between 5 µm*m⁻¹ (microstrain) and 6000 µm*m⁻¹ (microstrain) upon application of a load by the external component.

The first region may be configured as an annular region, and the second region may be a central region within the annular region. In some cases the second region extends through the entire first region. In some cases the second region extends through a portion of the first region.

The first and second surfaces of the first region may be at opposed sides of the first region. The second region may have a first surface arranged to engage the first bone surface. The second region may have a second surface arranged to engage the second bone surface.

The elastic modulus of the second region may be less than the elastic modulus of the first region.

This is advantageous to encourage bone in-growth within the second region of the implant. The orthopaedic implant may be configured as a spinal fusion cage, in particular an Anterior Lumbar Interbody Fusion (ALIF) spinal cage or a Posterior Lumbar Interbody Fusion (PLIF) spinal fusion cage.

The lattice structure may comprise a plurality of interconnected struts. Each strut may have a cross-sectional dimension of between 0.1mm and 0.5mm.

The lattice structure may have a strut density of between 3.0 per mm³ and 5.0 struts per mm³. The lattice structure may have a strut density of approximately 4.2 per mm³

The lattice structure may comprise a plurality of nodes, wherein each node is connected to at least one another node by a respective strut. The average number of connections between the plurality of nodes may be between 4.5 and 6.0. The average number of connections between the plurality of nodes may be between 5.3.

The load may be transferred from the securing element to the engaging surface via the nodes and struts of the lattice structure. That is, struts may be loaded axially or in bending, and nodes may transfer axial or bending loads between struts. Struts and/or nodes may transfer loads to the bone surface. The magnitude and direction of the strain at any one position on the engaging surface may be controlled by the properties of the lattice structure. For example. the average number of nodes, the orientation of the struts, the cross-sectional dimensions of the struts and/or the strut density, in the lattice structure.

The lattice structure may have an anisotropic elastic modulus.

Providing a range of cross-sectional dimensions, strut densities and node connections in the lattice structure is advantageous as it allows the elastic modulus and directionality of the lattice structure to be tuned as required by a particular patient or surgical procedure. Providing anisotropy is advantageous as the lattice structure is better matched to the mechanical properties of the underlying bone.

The width of the intermediate region in the first direction may be less than the width of the first and second regions in the first direction.

The lattice structure may make up at least 5% of the implant by volume. For example, the lattice structure may make up at least 10%, 15%, 20%, 25%, 30%, 35% or 40% or more of the implant by volume.

There is also provided a computer program element comprising and/or describing and/or defining a three-dimensional design of the orthopaedic implant described above. The three-dimensional design may be for use with a simulation means or an additive or subtractive manufacturing means, system or device. The orthopaedic implant is preferably made using an additive manufacturing technique, such as laser sintering.

There is also disclosed a method for manufacturing an orthopaedic implant comprising the steps of defining a three-dimensional design of an orthopaedic implant as described herein, transmitting the three-dimensional design to an apparatus for producing the orthopaedic implant, and causing the apparatus to produce the orthopaedic implant according to the three-dimensional design using an additive manufacturing technique.

Within the scope of this application it is expressly intended that the various aspects, embodiments, examples and alternatives set out in the preceding paragraphs, and/or in the following description and drawings, and in particular the individual features thereof, may be taken independently or in any combination. That is, all embodiments and/or features of any embodiment can be combined in any way and/or combination, unless such features are incompatible. For the avoidance of doubt, the terms "may", "and/or", "e.g.", "for example" and any similar term as used herein should be interpreted as non-limiting such that any feature so-described need not be present. Indeed, any combination of optional features is expressly envisaged without departing from the scope of the invention, whether or not these are expressly claimed.

### BRIEF DESCRIPTION OF THE DRAWINGS

The orthopaedic implant will now be described by way of example only with reference to the accompanying drawings in which:
Figure 1 is a schematic representation of a tibial component;
Figure 2 is an isometric view of a lower surface and keel of a tibial component of Figure 1;
Figure 3 is a detailed view of the lattice structure of the orthopaedic implant;
Figures 4a and 4b are schematic representations of a tibial component divided into regions;
Figures 5a and 5b are schematic representations of a tibial component with graded intermediate regions;
Figure 6 is a schematic representation of a tibial component with reinforcing regions; Figure 7 is a schematic representation of an exemplary femoral component according to the present disclosure;
Figures 8a and 8b illustrate plan and side views of a schematic representation of a spinal fusion cage not forming part of the claimed invention but useful for understanding the scope of the invention;
Figures 9a and 9b illustrate plan and side views of a schematic representation of a further exemplary spinal fusion cage not forming part of the claimed invention but useful for understanding the scope of the invention;
Figure 10 is a section view of an exemplary acetabular component according to the present disclosure;
Figure 11 is a graph showing interfacial strain differences between an orthopaedic implant and bone versus the difference in bulk modulus of the lattice and healthy bone;
Figure 12 is a graph showing interfacial stress differences between an orthopaedic implant and bone versus the difference in bulk modulus of the lattice and healthy bone;
Figure 13 is a graph comparing the maximum contact stress for implants according to the present disclosure, conventional implants and native bone; and
Figure 14 is a graph comparing the distribution of contact stress for implants according to the present disclosure, conventional implants and native bone.

### DETAILED DESCRIPTION

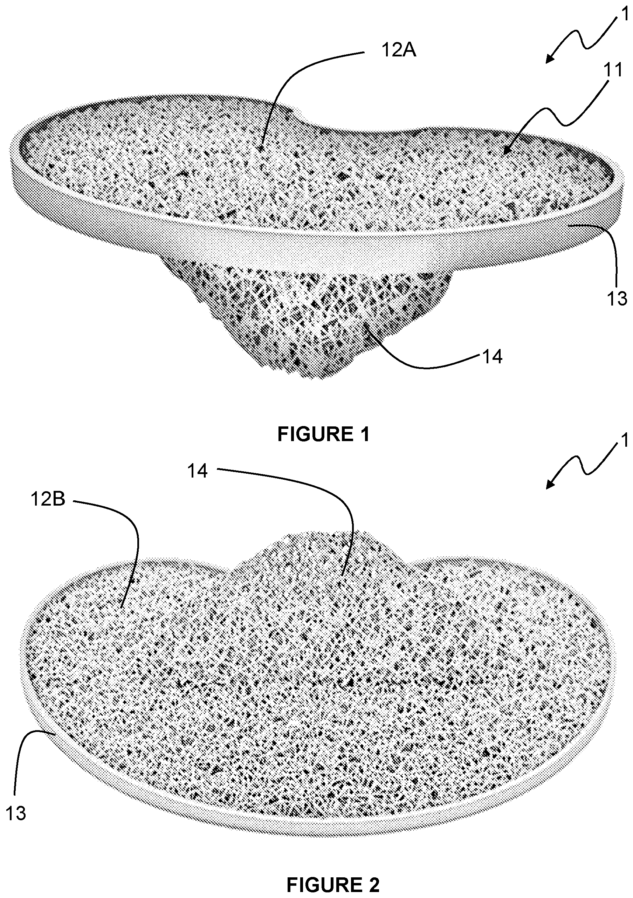

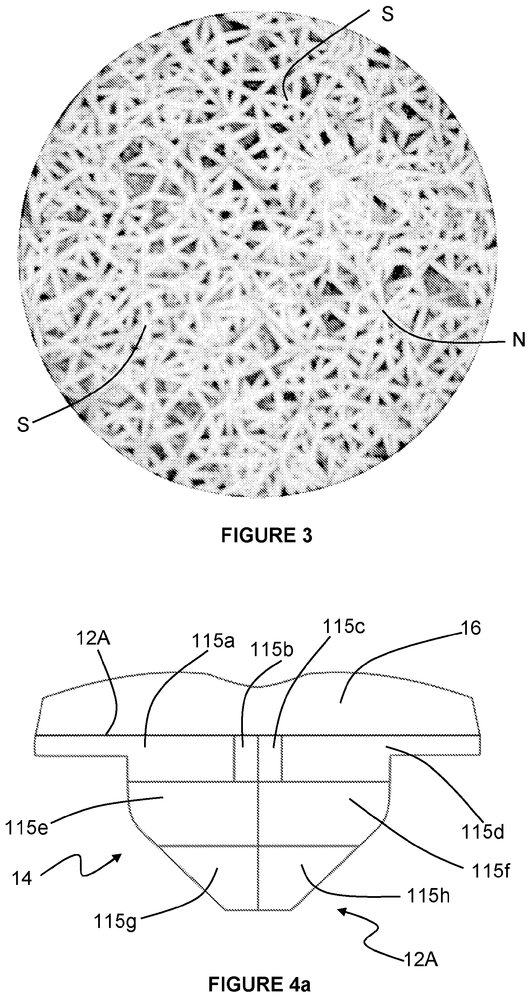

Referring now to Figures 1 and 2, there is shown an exemplary tibial component 1. The tibial component 1 has a lattice structure 11 shaped to provide the structural elements of a modular tibial component and includes an upper surface 12A, a lower surface 12B and a keel 14 protruding from the lower surface 12B. The keel 14 is shaped as a conventional keel in a tibial component and its function is to anchor the tibial component 1 to the prepared bone surface. The tibial component 1 also includes a band 13 for releasably connecting to a polymer tibial tray 16 (see Figure 4a) which acts as the articulating surface of the tibial component 1. This provides a convenient way for the surgeon to size the correct tibial component for a patient before assembling the final implant and implanting the chosen tibial component 1 into the prepared bone surface. The tibial tray 16 is one example of an external component that can be secured to the lattice structure 11. It would be apparent that the tibial tray 16 is shaped to engage a corresponding femoral component to provide an articulating surface once implanted.

As the band 13 is secured to the lattice structure 11, loads applied to the polymer tibial tray 16, for example those experienced during walking, siting, standing or other activities of daily living, will be transmitted to the lattice structure 11. Under normal loading conditions, for example under loads between 0.5 and 2 times the bodyweight of the subject, the tibial tray 16 transmits load from the femoral component to the lattice structure 11 and the underlying bone. Due to the increased deformability of the lattice structure 11 compared to solid titanium implants of the prior art, loads applied to the tibial tray 16 will result in increased deformation of the keel 14 and lower surface 12B, which, in turn, result in greater strain in the bone in contact with the tibial component 1. The increased levels of strain applied to the bone surface help to prevent bone loss, for example by encouraging bone modelling, which encourages the bone to adapt and grow into the tibial component 1 through the lattice structure 11. This provides a secure fixation between the implant and the underlying bone. As shown in Figure 1, the band 13 extends around a perimeter of the upper surface 12A, and as the lattice is deformed under loading of the tibial tray 16, this will cause the tibial tray 16 to contact the upper surface 12A, which will result in load being transmitted directly from the tibial component to the lattice structure 11, as well as indirectly via the band 13. Alternatively, or additionally, to the band 13, the lattice may include a securing part (not shown) secured to the upper portion of the lattice structure 11. The securing part may comprise a polymer material. The securing part may be cast into the lattice surface, for example up to approximately 5 mm into the lattice. In some cases the securing part may be cast between 2 to 3 mm into the upper surface of the lattice structure 11.

While a solid titanium band is shown, it would be apparent that this was merely one example of a suitable securing element for securing the tibial tray 16, and that other securing elements would be suitable. The keel 14 has a tapered profile which narrows with distance from the lower surface 12B. However, it would be apparent that this was merely exemplary and that other profiles of the keel 14 will be known in the art.

As shown in the Figures, the band 13 protrudes above the upper surface 12A and provides an opening into which a part of the tibial tray 16 can be received so as to secure the tibial tray 16 to the tibial component 1. The tibial tray 16 is of the type known in the art and is shaped to receive the femoral condyles of a corresponding femoral component. The band 13 is arranged to fix the tibial tray 16 relative to the tibial component 1. Any suitable method of fixation is envisaged to connect the tibial tray 16 to the band 13. By way of example, inter-engaging components formed on the band and the tibial tray 16 or a pressure fit connection may be used to secure the tibial tray 16 to the band 13.

Whilst in this example the tibial tray 16 is secured to the lattice structure 11, in some cases the tibial tray 16 may not be in direct contact with the upper surface 12A of the lattice structure 11. Instead the tibial tray 16 may be secured by the band 13, such that load is transmitted to the lattice structure 11 from the tibial tray 16, via the band 13 only. In this configuration, the lattice structure 11 and band 13 can be configured such that strain installed in the bone surrounding the tibial component 1 is similar to that installed in the surrounding bone when the tibial tray 16 is in direct contact with the lattice structure 11.

As shown in Figure 3 the lattice structure 11 comprises a plurality of struts S and a plurality of nodes N. Each node N is connected to at least one other node N by a respective strut S. The number of struts connected to a given node is defined as the connectivity of a node. In the illustrated example, the average number of connections between the plurality of nodes N is preferably between 4.5 and 6.0. However, it would be apparent that this was merely exemplary, and that other ranges of connectivity would be suitable in other applications, such as implants located at other joints, or in cases of especially stiff or compliant bone.

In the illustrated example, each strut S preferably has a cross-sectional dimension of between 0.1mm and 0.5mm. In the illustrated example, the lattice structure 11 has a strut density per unit volume of between 3 struts per mm³ and 5 struts per mm³. However, it would be apparent that these were merely exemplary values and that other values can be used to suit a range of patients.

The strut thickness, node connectivity and strut density per unit volume are parameters that can be used to tune the mechanical properties lattice structure 11 to achieve the desired levels of strain at the lower surface 12B and keel 14 which interface directly with the bone. One way of achieving this is to tune the stiffness of regions within the lattice structure 11 such that the lattice structure 11 has pre-determined bulk, or macro, mechanical properties. For example, a lattice structure 11 having a bulk elastic modulus between 0.1GPa and 20GPa has been found to provide sufficient mechanical strain at both the bone interface surfaces and also in the underlying bone to induce bone in-growth into the lattice structure 11.

A further parameter that can be used to tune the mechanical properties of the lattice structure 11 are the angles of the struts relative to, for example, a longitudinal axis of the keel 14. By angling the struts relative to the longitudinal axis of the keel 14, it is possible to provide a the lattice structure 11 with an anisotropic elastic modulus. The anisotropy of the bulk elastic moduli of the lattice structure 11 can be selected to correspond to the anisotropy, or the ratio of directional values of elastic modulus at a single point, of bone material at the superior end of the tibia to provide an optimised match between the tibial component 1 and the patient-specific bone properties. In the case of the illustrated tibial component 1, the longitudinal axis extends in a superior-inferior direction from the lower surface 12B. The upper 12A and lower 12B surfaces are substantially parallel to one another, and are preferably located within the band 13. However, it would be apparent that this was not essential and that the upper 12A and lower 12B surfaces may be arranged differently depending on surgical requirements and/or to suit a range of patients.

The strut thickness, node connectivity, strut density per unit volume and the angles of the struts are hereinafter collectively referred to as the lattice properties of the lattice structure 11.

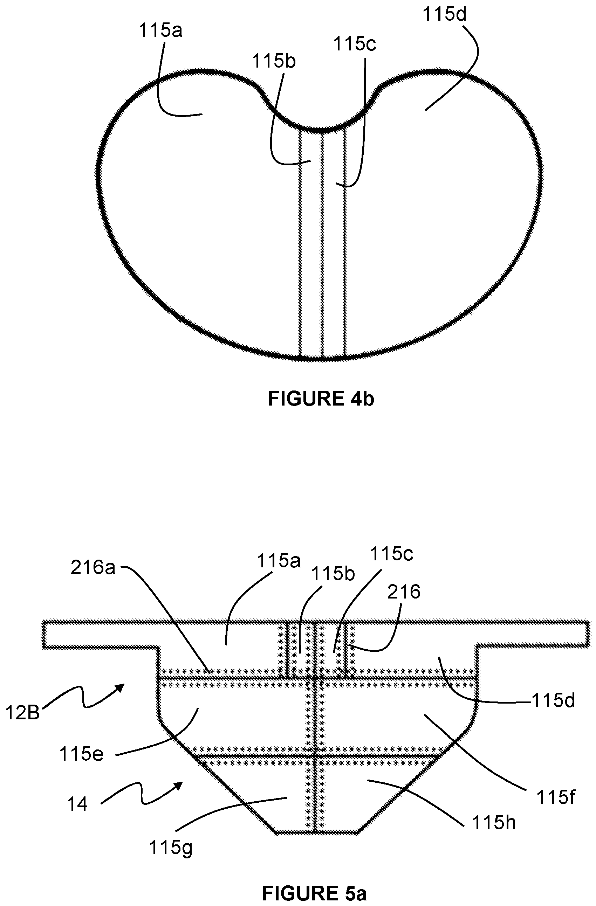

As shown in Figures 4a and 4b, the lattice structure 11 can be divided into eight regions 115a-h. Figure 4a shows a side view of the tibial component 1, with the tibial tray 16 attached, and Figure 4b shows a top view of the tibial component 1, with the tibial tray 16 omitted. Each region 115a-h of the lattice structure 11 has a uniform elastic modulus, and in the illustrated example, each region 115a-115h has different elastic modulus to the other regions. It would be apparent that the lattice structure 11 could be divided into more or fewer than eight regions, and that eight regions was merely an example of how the lattice structure 11 could be divided. Similarly, while each region is described as having a different modulus to all other regions in the tibial component 1, it would be apparent this was not essential. The elastic modulus of an individual region 115 refers to the bulk properties of the individual region 115, rather than the overall properties of the lattice structure 11. It would be apparent that each region 115 can be tuned such that any sub-portion of a region 115 would have an average modulus within a pre-determined amount, for example within 10%, of the bulk modulus of the overall region 115. Thus, adjacent regions 115 can have distinct moduli, and therefore exhibit distinct levels of strain, and the overall lattice structure 11 can be tuned to have the necessary strength and provide appropriate levels of deformation at the engaging surface.

In the illustrated tibial component 1 each region 115a-h has an elastic modulus according to Table 1.

**TABLE 1: Elastic moduli of regions in Figure 4.**

| **Region** | **Elastic modulus (GPa)** |
|---|---|
| 115a | 0.61 |
| 115b | 0.59 |
| 115c | 0.58 |
| 115d | 0.56 |
| 115e | 0.57 |
| 115f | 0.34 |
| 115g | 0.53 |
| 115h | 0.29 |

By matching the elastic moduli of the regions 115a-h to those of healthy bone, the strain installed in the bone surrounding the tibial component 1 is more similar to healthy bone, which helps to encourage bone in-growth into the tibial component 1. As shown in Table 1 and Figure 4a, the stiffness of the tibial component 1 can decrease in an inferior direction.

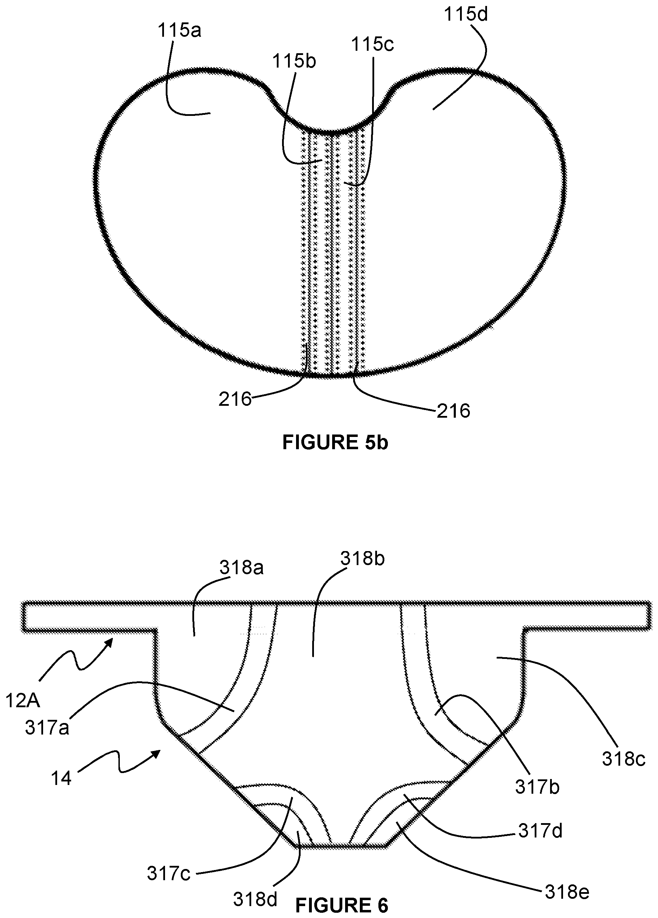

Figures 5a and 5b show the tibial component 1 of Figures 4a and 4b with intermediate regions 216 provided between adjacent pairs of regions 115a-h, as illustrated by dashed lines in Figures 5a and 5b.

The bulk elastic modulus in each intermediate region 216 is graded between the two regions either side of the intermediate region 216. The bulk elastic modulus in each intermediate region 216 is graded from the bulk elastic modulus of the region on one side, to the bulk elastic modulus of the region on the other side. Thus, the intermediate region 216 will have an elastic modulus that primarily varies in a single direction. It would be apparent that where multiple intermediate regions 216 merge, the elastic modulus may vary along multiple direction to provide a gradual change in modulus from one region 115a-h having a bulk elastic modulus to another region 115a-h having a different bulk elastic modulus. For example, region 115a has a bulk modulus of 0.61GPa and region 115e has a bulk modulus of 0.57GPa and the intermediate region 216a between the two regions would have a bulk modulus that varied from 0.61GPa where region 216a contacts region 115a, to 0.57GPa where region 216a contacts region 115e. Across the width of region 216a, the modulus varies in a linear manner. However, while a linear grading is described herein as an example of how one can grade the modulus across the intermediate region, it would be apparent this was merely exemplary and that other methods of grading the modulus would also apply. The intermediate region 216a may be considered to have multiple sub-portions with different average moduli to provide the overall graded modulus of the intermediate region 216a. As explained above, each sub-region may have further sub-regions contained within which have a modulus that is within a pre-determined amount, for example 10%, of the modulus of each sub-region, in order to provide the graded modulus.

By grading the strain at the lower surface 12B and keel 14 of the implant this leads to gradual changes in strain in the bone material, compared to a discrete step change in modulus between adjacent regions. The width of the intermediate regions 216 may be increased such that the strain over large sections of the lattice structure 11 are graded, to provide graded strain in the bone material more akin to the loading conditions of healthy bone. While the intermediate regions 216 are shown having the same uniform width, it would be apparent this was merely exemplary and that in some cases the intermediate regions 216 may have different widths to one another and any of the intermediate regions 216 may have a varying width across its length.

The tibial component 1 can be made using additive manufacturing techniques in order to provide the necessary fine control over the micro- and macro-material properties of the tibial component 1. For example, the same underlying design of tibial component can be tuned in one manner to provide a suitable implant for a patient having relatively stiff bone, and tuned in a different manner to provide a suitable implant for a second patient having relatively compliant bone. This is a significant advantage of the present implant, as tuning the implant design, as opposed to re-designing an entire implant to account for patient specific characteristics, considerably reduces the time required to prepare and produce user-specific implants. One way to define the lattice structure 11 is to create an array of connected voxels having user-defined lattice properties. In this case, it is possible to grade each intermediate region 216 by incrementally changing the bulk elastic modulus of each voxel of the intermediate region 216, in the grading direction. The grading is typically linear across the intermediate region 216 and each voxel may have a different stiffness to an adjacent voxel within the same intermediate region 216 when going from the first region to the second region.

It will be appreciated that the properties of each intermediate regions 216 can be defined to the level of a single voxel. For example, where the minimum voxel dimension is 3mm, this corresponds to an intermediate region 216 having a minimum width of 3 mm. In this case, the bulk mechanical properties of the intermediate regions 216 will be the mean average of the two adjacent regions. Similarly, the intermediate regions 216 can be significantly wider than those represented in Figure 5a.

Figure 6 illustrates the tibial component 1 where the lattice structure 11 incorporates four reinforcing regions 317a-d located between regions 318a-e. The bulk elastic moduli of the reinforcing regions 317a-d is greater than the bulk elastic moduli of the regions 318a-e, as the reinforcing regions 317a-d provide additional structural integrity to the lattice structure 11. It should be noted that in some cases, the reinforcing regions 317 may be higher than 20GPa and that reference to the implant having a bulk elastic modulus is exclusive of any reinforcing regions 317. These reinforcing regions 317a-d may be used in a tibial component for a patient with particularly weak and compliant bone, as the desired levels of strain may be achieved with relatively thin struts. As such, it is desirable to include the reinforcing regions 317a-d so that the deformable bone engaging surfaces 12B, 14 are adequately supported, as failure of the tibial component 1 would require revision surgery which is highly undesirable. The bulk regions 318a-e are preferably tuned in a similar manner described above to match the properties of the bone into which the tibial component 1 is implanted. Similarly, the reinforcing regions 317a-d may each have different elastic moduli or the same elastic moduli. It would also be apparent that while four arcuate reinforcing regions 317a-d are shown, it would be apparent that one or more of the reinforcing regions can have a different profile (such as a linear profile), and that more than four or fewer than four reinforcing regions may be incorporated. Similarly, while the reinforcing regions are shown extending from the upper surface 12A to the keel 14 and between different parts of the keel 14, it would be apparent that this was not essential.

The lattice structure 11 is preferably manufactured using titanium, although it would be apparent that a lattice structure 11 made from other materials would benefit from the present disclosure. While the tibial component 1 is preferably manufactured using an additive manufacturing technique, such as laser sintering, it would be apparent that other additive manufacturing techniques would also be suitable. Each strut S is preferably manufactured using an exposure time of 100 microsecond to 800 microseconds, to achieve the desired strut thickness.

By manufacturing the lattice structure 11 of the tibial component 1 with bulk elastic moduli properties between 0.1 GPa and 20GPa, it is possible to tune the lattice structure 11 to account for varying bone quality between patients as described above. For example a patient with more compliant bone may utilise a relatively compliant tibial component 1, possibly incorporating reinforcing regions, compared to a patient with relatively stiff bone, while the resulting levels of strain at the bone interface and in the underlying bone of both patients may be at the same desired levels of strain that promote bone in-growth, for example between 50 and 3000 µm*m⁻¹ (microstrain). These levels of strain are up to 15% of the normal levels of strain experienced by healthy bone, which encourages bone in-growth into the lattice structure 11. This level of strain also far exceeds those exhibited by solid titanium implants. This also installs strains in the surrounding bone between 1500 µm*m⁻¹ (microstrain) and 3500 µm*m⁻¹ (microstrain), which leads to modelling of the bone material in these regions. This prevents bone loss in the bone material surrounding the implant, and so reduces premature aging and resorption of the bone material surrounding the tibial component 1, which would otherwise lead to implant loosening and possibly require revision surgery to correct.

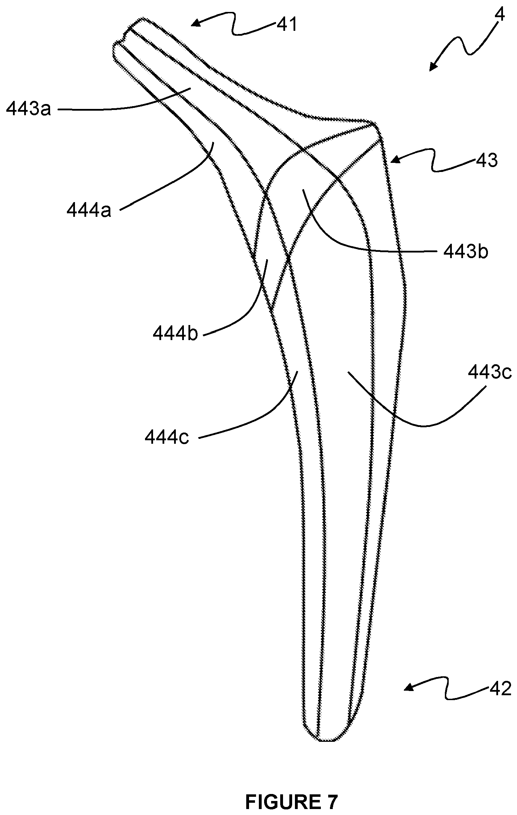

Figure 7 shows a femoral component 4 which includes a lattice structure 43 that can be tuned in the same manner as described above in relation to lattice structure 11. The mechanical properties, grading aspects, reinforcing aspects described in relation to lattice structure 11 apply equally to lattice structure 43. The lattice structure 43 has a proximal end 41 for connecting to a femoral head component and a distal end 42 for insertion into the femur of a patient. The outer surface of any of the regions 444b, 443c and 444c may act as a bone-engaging surface with the prepared femoral bone surface.

The lattice structure 43 is shown divided into six regions in Figure 7. The lattice structure 43 is divided into three central regions 443a-c, and each central region 443a-c is shown surrounded by a respective annular region 444a-c. Each region 443a-c, 444a-c of the femoral component 44 has a uniform elastic modulus which can be tuned as required to best match the patient's bone. While the annular regions are described as surrounding a respective central region, it would be apparent that the annular region could be made up of a plurality of regions, each having a different elastic modulus.

The bulk elastic modulus of the regions 443a-c, 444a-c of the femoral component 44 are shown in Table 2. The bulk elastic moduli are distributed to provide femoral component 4 having a stiffer core and a more compliant periphery which is in contact with bone surface.

**TABLE 2: Elastic moduli of regions in Figure 7.**

| **Region** | **Elastic modulus (GPa)** |
|---|---|
| 443a | 0.62 |
| 443b | 0.90 |
| 443c | 0.30 |
| 444a | 0.10 |
| 444b | 0.54 |
| 444c | 0.30 |

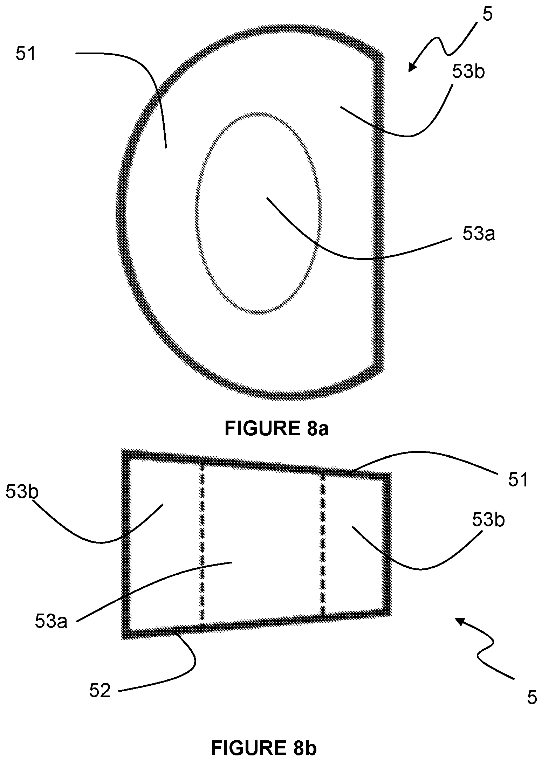

Figures 8a and 8b show a spinal fusion cage 5 incorporating a lattice structure. The spinal fusion cage 5 is configured as an anterior lumbar interbody fusion (ALIF) spinal cage has a first surface 51 arranged to engage a bone surface of a first vertebra and a second surface 52 arranged to engage a bone surface of a second vertebra adjacent the first vertebra. The spinal fusion cage 5 is shown partially elliptical in plan, having a flat side parallel to the major diameter of the ellipse. The spinal fusion cage 5 is a parallel extrusion of the partial elliptical shape. However, it would be apparent this was merely exemplary, and that other shapes would be suitable. The contact surfaces 51, 52 form the ends of the extrusion. The contact surfaces 51, 52 are substantially planar, and arranged such that the two planes of the contact surfaces 51, 52 form an acute angle therebetween at the flat side of the partial ellipse shape.

The parameters and bulk mechanical properties used to define the lattice structure of the tibial 1 and femoral 4 components above apply equally to the lattice structure of the spinal fusion cage 5.

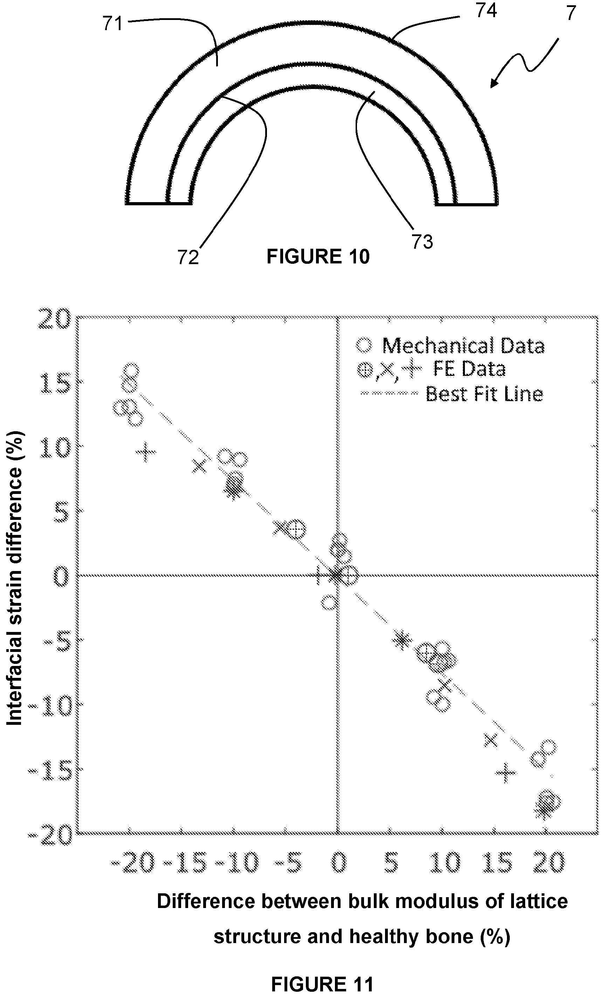

The lattice structure of spinal fusion cage 5 is divided into a central region 53a and an annular region 53b. The outer region 53b surrounds the central region 53a, and the central region 53a extends along the entire spinal fusion cage 5 as shown by the dashed lines in Figure 10b. The mechanical properties, grading aspects, reinforcing aspects described in relation to lattice structure 11 apply equally to lattice structure to the central region 53a and annual region 53b of the spinal fusion cage 5. While the central region 53a is shown extending through the depth of the spinal fusion cage 5, it would be apparent that this was not essential and that in some cases, the central region 53a may be contained entirely within the annular region 53b such that only the annular region 53b contacts the adjacent bone surfaces.

The bulk elastic modulus of the annular region 53b is greater than the bulk elastic modulus of the central region 53a. Specifically, in this example, the central region 53a has a bulk elastic modulus of approximately 0.1GPa, and the outer region 53b has a bulk elastic modulus of approximately 2.5GPa. However, it would be apparent that this was not essential and that the modulus of the of the annular 53b and central 53a regions may be varied to suit a range of patients.

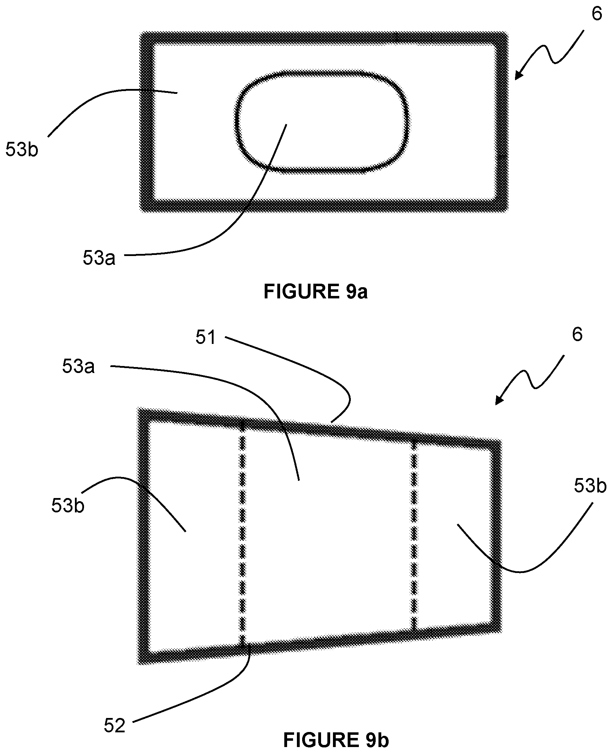

Figures 9a and 9b show a further exemplary spinal fusion cage 6. The spinal fusion cage 6 is a Posterior Lumbar Interbody Fusion (PLIF) spinal fusion cage and a similar structure to the spinal fusion cage 5 and corresponding features are numbered similarly.

Figure 10 shows a schematic section view through a central plane of an exemplary acetabular cup 7 incorporating a lattice structure 71. The lattice structure 71 can be tuned in the same way as the lattice structures of the tibial component 1 and the femoral component 4, as previously described. The mechanical properties, grading aspects, reinforcing aspects described in relation to lattice structures 11, 43 apply equally to lattice structure 71. The lattice structure 71 has a radially inner surface 72 for connecting to an articulating component 73. The articulating component 73 is configured to receive a femoral component, for example a femoral head which corresponds to the geometry of the articulating component 73. The lattice structure 71 has a radially outer surface 74 for insertion into the acetabulum of a patient. The radially outer surface 74 engages the prepared bone surface of the acetabulum to fix the implant 7 to the pelvis of the patient. The articulating component 73 preferably includes a polymer articulating surface for engaging the femoral head.

It will be appreciated by those skilled in the art that the illustrated implants show tibial, femoral and acetabular components, and spinal fusion cages, all for use in humans. However, implants for animals would also benefit from the present disclosure.

Exemplary data are now provided to further illustrate the advantages of the present implants.

As explained previously, reducing the bulk elastic modulus of the lattice to resemble that of healthy bone in the volume replaced by the lattice structure, reduces the difference in strain at the interface between the bone and the lattice structure, when the implant is in use.

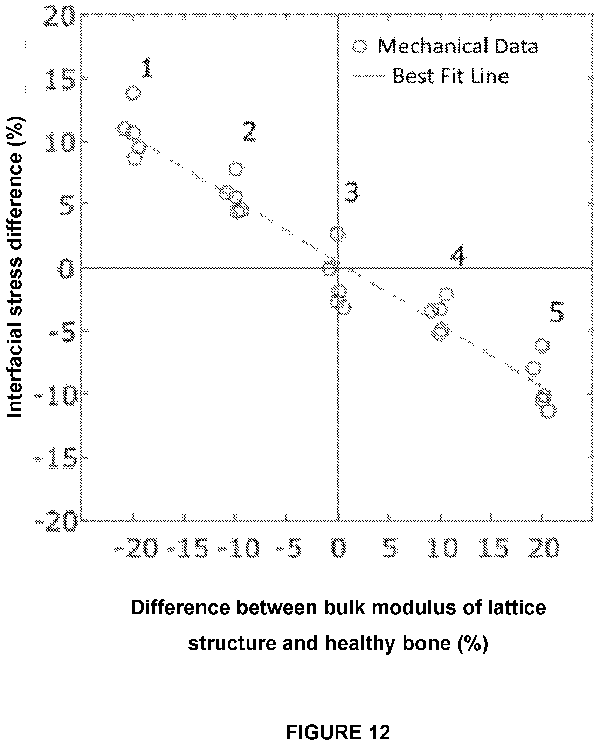

Figures 11 and 12 show experimental test results, referred to as 'Mechanical Data' and finite element analysis results, referred to as 'FE Data' for lattice samples with various bulk modulus values relative to that of the tested bone sample.

Figure 11 shows that, when the bulk modulus of the lattice structure is within +/- 20% of that of healthy bone, the difference between the strain in the lattice structure, and the strain in the bone, at the interface between the bone and the lattice structure (referred to as the 'Interfacial strain difference' in Figure 10), is also within the range of +/-20%. Specifically, when the lattice is stiffer than healthy bone, there is a corresponding decrease in the interfacial strain, and when the lattice is more compliant than healthy bone, there is a corresponding increase in interfacial strain. Thus, it is possible to obtain a desired level of strain by tuning the stiffness of the lattice by selecting appropriate lattice properties.

Figure 12 shows that, when the bulk modulus is within +/- 20% of that of bone, the difference between the stress in the lattice structure, and the stress in the bone, at the interface between the bone and the lattice structure (referred to as the 'Interfacial stress difference' in Figure 11), is within the range of +/-20%. The numbers inside the plot area of Figure 11 indicate test sample number. Specifically, when the lattice is stiffer than healthy bone, there is a corresponding decrease in interfacial stress, and when the lattice is more compliant than healthy bone, there is a corresponding increase in interfacial stress. Thus, it is possible to obtain a desired level of interfacial stress by tuning the stiffness of the lattice by selecting appropriate lattice properties also.

This data shows that the modulus of the lattice structure must only match that of the bone material within +/-20% in order to match the strains to within +/-15%. This shows that, in some cases, patient specific data is not required to achieve interfacial strain difference which encourage in-growth of bone into the lattice structure. This is a further advantage of the present implants, as components can be manufactured for a wide range of patients without knowing anything about the patients, while still delivering the benefits of improved bone in-growth. For example, where an implant is for a patient with bone stiffness of a pre-determined value, the lattice structure may have a bulk stiffness of 0.8 of the pre-determined value. For example, where an implant is for a patient with bone stiffness of a pre-determined value, the lattice structure may have a bulk stiffness of 1.2 of the pre-determined value. It would be apparent that the pre-determined value may be taken as an estimate for the given patient based on empirical calculations based on a patient metric, such as age, height, weight, limb length, or any other patient metric described herein.

As also explained previously, by transferring load directly through the lattice structure of the orthopaedic implant, the contact pressures between the implant and the surrounding bone material are increased to levels similar to the stresses experienced by healthy bone. This is because load is not bypassed though high stiffness parts of the implant due to the stress shielding effects exhibited by solid implants.

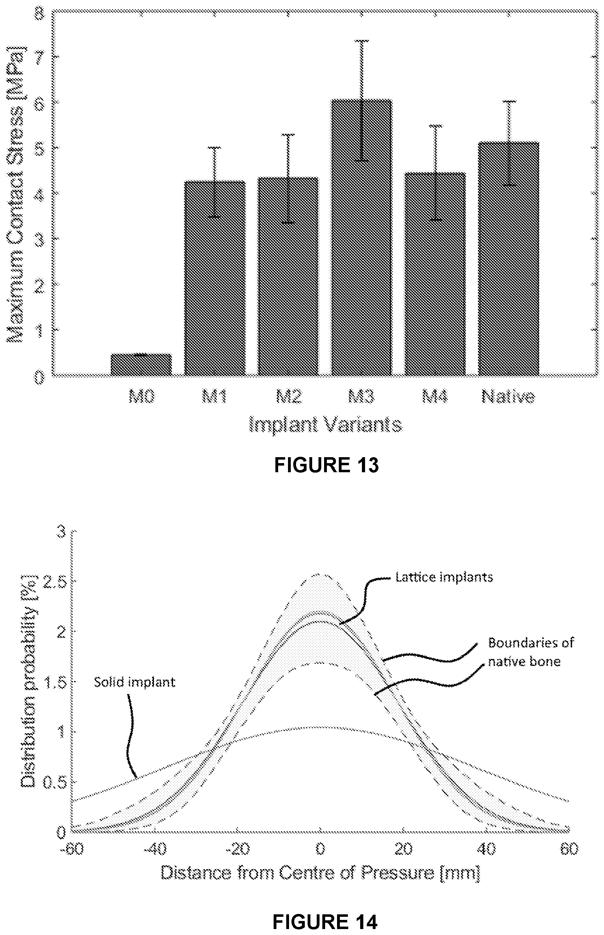

Figure 13 shows the maximum contact stress between tibial implants according to the present disclosure (labelled as 'M1', 'M2', 'M3' and 'M4' in Figure 12), and bone material. Also shown is the maximum contact stress between a solid titanium implant (labelled as 'M0' in Figure 12) and bone material. These values are compared to the maximum stress experienced in healthy bone (labelled as 'Native' in Figure 12) in the areas in which the contact stress arises in bone with the implants present.

It can be seen that the maximum contact stress in the solid titanium implant, M0, is much less than that in native bone, due to the even distribution of load across the implant surface. On the contrary, the maximum contact stresses in the lattice implants, M1-4, are similar to the stress in native bone, as the present implants more closely resemble the stiffness distribution of native bone which has pressure points in the load distribution across the implant surface.

Therefore, it can be seen that conventional solid titanium implants install much less strain in the surrounding bone material than an implant according to the present disclosure.

Figure 14 shows the distribution of stress around the highest contact stress point, for the test data illustrated in Figure 13. It can be seen that the present implants distribute stress in a much more similar manner to that experienced in native bone, whereas the contact stresses in the solid implant are much more distributed across the entire implant surface. This further illustrates that the strains installed in the bone material surrounding, and spaced from, implants according to this invention are much greater than those of conventional implants.

It would be apparent that the load applied to the articulating joint component could be in any direction. In the case of a tibial or femoral knee component, the majority of the load will typically be applied along a superior-inferior direction of the tibial component or the femoral knee component respectively. However, this is not essential, particularly as the knee flexes, the loading direction relative to the respective articulating surfaces may change in a corresponding manner. As the implant is mechanically engaged with the surrounding bone, the strain installed into the tissue in contact with the engaging surface will also typically be in the same direction as the direction of load applied to the articulating component. As bone is a continuous structure, it would be apparent that application of a load to the bone in one direction would not necessarily result in deformation limited to the same direction. It is likely that the deformation of the engaging surface in one direction will cause deformation within the bone in a number of directions. Such engagement results in greater stimulation within the bone which further enhances the fixation between the implant and the underlying bone.

For example, a load applied at a contact point on the articulating component can be applied in a normal direction to a plane which is tangential to the contact point the articulating component. In this case, the engaging surface will deform primarily in a normal direction to the contact point. However, as explained above, a shear strain will be applied to the bone tissue engaged with the engaging surface and adjacent to the contact point. It would also be apparent that the deformation of the engaging surface may result in a combination of normal strain and shear strain.

In some cases, the load applied in a perpendicular direction at a contact point on the articulating component. The contact point may be where the articulating component abuts a corresponding part. For example, a tibial component is designed to engage a femoral part having a corresponding articulating surface. An acetabular cup is designed to correspond with a femoral head having a corresponding articulating surface. The engaging surface can apply a strain to the surrounding tissue by engaging the soft tissue.

The application of load by the articulating joint component may be between 0.5 and 2 times bodyweight of a patient. It would be apparent that an implant will be selected based on a number of patient metrics, including bodyweight. Deformation of the engaging surface installs strain in the bone surface, or in bone material underlying the bone surface, in use, which differs from the strain in the adjacent engaging surface by less than +/- 20%, for example by less than +/- 15%.

One or more load paths may be defined within the lattice structure. For example, a first load path may extend between the securing element and the engaging surface. A second load path may extend between the surface abutting the articulating component and the engaging surface. The elastic modulus of the lattice structure may vary with position in the lattice structure, such that a load transferred along any lattice load path results in a strain of between 5 µm*m⁻¹ (microstrain) and 6000 µm*m⁻¹ (microstrain) on the engaging surface.

## Claims

1. An orthopaedic implant (1, 4, 7) comprising:
a securing element (13) configured to engage an articulating joint component (16, 73); and
a lattice structure (11, 43, 71) connected to the securing element (13) and having an engaging surface (74) configured, in use, to engage a bone surface,
wherein the securing element (13) provides a load path between the articulating joint component (16, 73) and the lattice structure (11, 43, 71) when the securing element is connected to the articulating joint component,
wherein the engaging surface (74) is configured to deform upon application of a load by the articulating joint component (16, 73), and wherein the lattice structure (11, 43, 71) has a second surface (12A, 72) arranged, in use, to abut the articulating joint component (16, 73), and
wherein the lattice structure (11, 43, 71) comprises:
a first region (115a-h, 318a-e, 443a-c, 444a-c) having a first elastic modulus;
a second region (115a-h, 318a-e, 443a-c, 444a-c) having a second elastic modulus different to the first elastic modulus; and
an intermediate region (216) between the first and second regions (115a-h, 318a-e, 443a-c, 444a-c),
wherein the intermediate region (216) has multiple sub-portions with different elastic moduli such that the elastic modulus of the intermediate region (216) is graded in a first direction extending from the first region (115a-h, 318a-e, 443a-c, 444a-c) to the second region (115a-h, 318a-e, 443a-c, 444a-c), such that the elastic modulus of the intermediate region (216) varies from the first elastic modulus to the second elastic modulus in the first direction;
**characterized in that** the lattice structure has an elastic modulus of between 0.1 GPa and 20 Gpa,
and **in that** the securing member comprises a band (13) arranged around a portion of the lattice structure (11).

2. An orthopaedic implant (1, 4, 7) according to claim 1, wherein the elastic modulus of the intermediate region (216) is graded linearly in the first direction.

3. An orthopaedic implant (1, 4, 7) according to claim 1 or 2, wherein the width of the intermediate region (216) in the first direction is less than the width of the first and second regions (115a-h, 318a-e, 443a-c, 444a-c) in the first direction, and/or wherein a portion of the intermediate region (216) between the first and second regions (115a-h, 318a-e, 443a-c, 444a-c) has a width of at least 3mm.

4. An orthopaedic implant (1, 4, 7) according to any preceding claim, wherein the elastic modulus of the lattice structure (11, 43, 71) adjacent the engaging surface (74) is less than the elastic modulus of a proximal portion of the lattice structure (11, 43, 71) spaced from the engaging surface (74).

5. An orthopaedic implant (1, 4, 7) according to any preceding claim, wherein the lattice structure (11, 43, 71) comprises a central portion (115b-c, 443a-c) and a peripheral portion (115a, 115d-h, 444a-c), and wherein the elastic modulus of the peripheral portion (115a, 115d-h, 444a-c) is less than the elastic modulus of the central portion (115b-c, 443a-c).

6. An orthopaedic implant (1, 4, 7) according to any preceding claim, wherein the elastic modulus of the securing member (13) is greater than the elastic modulus of the lattice structure (11, 43, 71).

7. An orthopaedic implant (1, 4, 7) according to any preceding claim, wherein the lattice structure (11, 43, 71) has an elastic modulus between 3 GPa and 20 GPa.

8. An orthopaedic implant (1, 4, 7) according to any preceding claim, wherein the articulating joint component is a polymer articulating joint component (16, 73).

9. An orthopaedic implant (1, 4, 7) according to any preceding claim, wherein the orthopaedic implant (1, 4, 7) is any of a modular knee implant or a modular hip implant and/or wherein the orthopaedic implant (1, 4, 7) is configured as any of a tibial component, a femoral component or an acetabular cup.

10. An orthopaedic implant (1, 4, 7) according to any preceding claim, wherein the lattice structure (11, 43, 71) comprises a plurality of interconnected struts, and wherein each strut has a cross-sectional dimension of between 0.1mm and 0.5mm; and/or wherein the lattice structure (11, 43, 71) has a strut density of between 3.0 per mm³ and 5.0 struts per mm³.

11. An orthopaedic implant (1, 4, 7) according to any preceding claim, wherein the lattice structure (11, 43, 71) comprises a plurality of nodes, wherein each node is connected to at least one another node by a respective strut, and wherein the average number of connections between the plurality of nodes is between 4.5 and 6.0.

12. An orthopaedic implant (1, 4, 7) according to any preceding claim, wherein the lattice structure (11, 43, 71) has an anisotropic elastic modulus.

13. An orthopaedic implant (1, 4, 7) according to any preceding claim, wherein the lattice structure (11, 43, 71) makes up at least 5% of the implant by volume.

14. An orthopaedic implant (1) according to any preceding claim, wherein the lattice structure (11) comprises a plurality of reinforcing regions (317a-d) having an elastic modulus greater than the first elastic modulus and the second elastic modulus.

## Patentansprüche

1. Orthopädisches Implantat (1, 4, 7), umfassend:
ein Sicherungselement (13), das konfiguriert ist, um in eine Gelenkverbindungskomponente (16, 73) einzugreifen; und
einer Gitterstruktur (11, 43, 71), die mit dem Sicherungselement (13) verbunden ist und eine Eingriffsfläche (74) aufweist, die konfiguriert ist, um in Verwendung in eine Knochenfläche einzugreifen,
wobei das Sicherungselement (13) einen Lastweg zwischen der Gelenkverbindungskomponente (16, 73) und der Gitterstruktur (11, 43, 71) bereitstellt, wenn das Sicherungselement mit der Gelenkverbindungskomponente verbunden ist,
wobei die Eingriffsfläche (74) konfiguriert ist, um sich bei Anwendung einer Last durch die Gelenkverbindungskomponente (16, 73) zu verformen, und wobei die Gitterstruktur (11, 43, 71) eine zweite Fläche (12A, 72) aufweist, die in Verwendung angeordnet ist, um an der Gelenkverbindungskomponente (16, 73) anzuliegen, und
wobei die Gitterstruktur (11, 43, 71) Folgendes umfasst:
einen ersten Bereich (115a-h, 318a-e, 443a-c, 444a-c), der einen ersten Elastizitätsmodul aufweist;
einen zweiten Bereich (115a-h, 318a-e, 443a-c, 444a-c), der einen zweiten Elastizitätsmodul aufweist, der sich von dem ersten Elastizitätsmodul unterscheidet; und
einen Zwischenbereich (216) zwischen dem ersten und dem zweiten Bereich (115a-h, 318a-e, 443a-c, 444a-c),
wobei der Zwischenbereich (216) mehrere Unterabschnitte mit unterschiedlichen Elastizitätsmodulen aufweist, sodass der Elastizitätsmodul des Zwischenbereichs (216) in einer ersten Richtung abgestuft ist, die sich von dem ersten Bereich (115a-h, 318a-e, 443a-c, 444a-c) zu dem zweiten Bereich (115a-h, 318a-e, 443a-c, 444a-c) erstreckt, sodass der Elastizitätsmodul des Zwischenbereichs (216) von dem ersten Elastizitätsmodul zu dem zweiten Elastizitätsmodul in der ersten Richtung variiert;
**dadurch gekennzeichnet, dass** die Gitterstruktur einen Elastizitätsmodul zwischen 0,1 GPa und 20 GPa aufweist, und dass
das Sicherungselement ein Band (13) umfasst, das um einen Abschnitt der Gitterstruktur (11) angeordnet ist.

2. Orthopädisches Implantat (1, 4, 7) nach Anspruch 1, wobei der Elastizitätsmodul des Zwischenbereichs (216) linear in der ersten Richtung abgestuft ist.

3. Orthopädisches Implantat (1, 4, 7) nach Anspruch 1 oder 2, wobei die Breite des Zwischenbereichs (216) in der ersten Richtung geringer als die Breite des ersten und des zweiten Bereichs (115a-h, 318a-e, 443a-c, 444a-c) in der ersten Richtung ist und/oder wobei ein Abschnitt des Zwischenbereichs (216) zwischen dem ersten und dem zweiten Bereich (115a-h, 318a-e, 443a-c, 444a-c) eine Breite von zumindest 3 mm aufweist.

4. Orthopädisches Implantat (1, 4, 7) nach einem vorhergehenden Anspruch, wobei der Elastizitätsmodul der Gitterstruktur (11, 43, 71) benachbart zu der Eingriffsfläche (74) geringer als der Elastizitätsmodul eines proximalen Abschnittes der Gitterstruktur (11, 43, 71) beabstandet von der Eingriffsfläche (74) ist.

5. Orthopädisches Implantat (1, 4, 7) nach einem vorhergehenden Anspruch, wobei die Gitterstruktur (11, 43, 71) einen zentralen Abschnitt (115b-c, 443a-c) und einen peripheren Abschnitt (115a, 115d-h, 444a-c) umfasst und wobei der Elastizitätsmodul des peripheren Abschnittes (115a, 115d-h, 444a-c) geringer als der Elastizitätsmodul des zentralen Abschnittes (115b-c, 443a-c) ist.

6. Orthopädisches Implantat (1, 4, 7) nach einem vorhergehenden Anspruch, wobei der Elastizitätsmodul des Sicherungselements (13) größer als der Elastizitätsmodul der Gitterstruktur (11, 43, 71) ist.

7. Orthopädisches Implantat (1, 4, 7) nach einem vorhergehenden Anspruch, wobei die Gitterstruktur (11, 43, 71) einen Elastizitätsmodul zwischen 3 GPa und 20 GPa aufweist.

8. Orthopädisches Implantat (1, 4, 7) nach einem vorhergehenden Anspruch, wobei die Gelenkverbindungskomponente eine Polymergelenkverbindungskomponente (16, 73) ist.

9. Orthopädisches Implantat (1, 4, 7) nach einem vorhergehenden Anspruch, wobei das orthopädische Implantat (1, 4, 7) ein beliebiges von einem modularen Knieimplantat oder einem modularen Hüftimplantat ist und/oder wobei das orthopädische Implantat (1, 4, 7) als ein beliebiges von einer Tibiakomponente, einer Femurkomponente oder einer Hüftpfanne konfiguriert ist.

10. Orthopädisches Implantat (1, 4, 7) nach einem vorhergehenden Anspruch, wobei die Gitterstruktur (11, 43, 71) eine Vielzahl von miteinander verbundenen Streben umfasst und wobei jede Strebe eine Querschnittsabmessung zwischen 0,1 mm und 0,5 mm aufweist; und/oder wobei die Gitterstruktur (11, 43, 71) eine Strebendichte zwischen 3,0 pro mm³und 5,0 Streben pro mm³ aufweist.

11. Orthopädisches Implantat (1, 4, 7) nach einem vorhergehenden Anspruch, wobei die Gitterstruktur (11, 43, 71) eine Vielzahl von Knoten umfasst, wobei jeder Knoten mit zumindest einem anderen Knoten durch eine jeweilige Strebe verbunden ist und wobei die durchschnittliche Anzahl an Verbindungen zwischen der Vielzahl von Knoten zwischen 4,5 und 6,0 ist.

12. Orthopädisches Implantat (1, 4, 7) nach einem vorhergehenden Anspruch, wobei die Gitterstruktur (11, 43, 71) einen anisotropen Elastizitätsmodul aufweist.

13. Orthopädisches Implantat (1, 4, 7) nach einem vorhergehenden Anspruch, wobei die Gitterstruktur (11, 43, 71) zumindest 5 Vol.-% des Implantats ausmacht.

14. Orthopädisches Implantat (1) nach einem vorhergehenden Anspruch, wobei die Gitterstruktur (11) eine Vielzahl von Verstärkungsbereichen (317a-d) umfasst, die einen Elastizitätsmodul aufweisen, der größer als der erste Elastizitätsmodul und der zweite Elastizitätsmodul ist.

## Revendications

1. Implant orthopédique (1, 4, 7) comprenant :
un élément de fixation (13) conçu pour se mettre en prise avec un composant de joint articulé (16, 73) ; et
une structure en treillis (11, 43, 71) reliée à l'élément de fixation (13) et comportant une surface de mise en prise (74) conçue, lors de l'utilisation, pour se mettre en prise avec une surface osseuse,
ledit élément de fixation (13) fournissant un trajet de charge entre l'élément de joint articulé (16, 73) et la structure en treillis (11, 43, 71) lorsque l'élément de fixation est relié à l'élément de joint articulé,
ladite surface de mise en prise (74) étant conçue pour se déformer lors de l'application d'une charge par l'élément de joint articulé (16, 73), et ladite structure en treillis (11, 43, 71) comportant une seconde surface (12A, 72) agencée, lors de l'utilisation, pour venir en appui contre l'élément de joint articulé (16, 73), et
ladite structure en treillis (11, 43, 71) comprenant :
une première zone (115a à h, 318a à e, 443a à c, 444a à c) comportant un premier module d'élasticité ;
une seconde zone (115a à h, 318a à e, 443a à c, 444a à c) comportant un second module d'élasticité différent du premier module d'élasticité ; et
une zone intermédiaire (216) entre les première et secondes zones (115a à h, 318a à e, 443a à c, 444a à c),
ladite zone intermédiaire (216) comportant de multiples sous-parties avec des modules d'élasticité différents de sorte que le module d'élasticité de la zone intermédiaire (216) soit gradué suivant une première direction s'étendant de la première zone (115a à h, 318a à e, 443a à c, 444a à c) à la seconde zone (115a à h, 318a à e, 443a à c, 444a à c), de sorte que le module d'élasticité de la zone intermédiaire (216) varie du premier module d'élasticité au second module d'élasticité suivant la première direction ;
**caractérisé en ce que** la structure en treillis comporte un module élastique compris entre 0,1 GPa et 20 Gpa, et **en ce que** l'élément de fixation comprend une bande (13) disposée autour d'une partie de la structure en treillis (11).

2. Implant orthopédique (1, 4, 7) selon la revendication 1, ledit module d'élasticité de la zone intermédiaire (216) étant gradué linéairement suivant la première direction.

3. Implant orthopédique (1, 4, 7) selon la revendication 1 ou 2, ladite largeur de la zone intermédiaire (216) suivant la première direction étant inférieure à la largeur des première et seconde zones (115a à h, 318a à e, 443a à c, 444a à c) suivant la première direction, et/ou une partie de la zone intermédiaire (216) entre la première et la seconde zone (115a à h, 318a à e, 443a à c, 444a à c) comportant une largeur d'au moins 3 mm.

4. Implant orthopédique (1, 4, 7) selon l'une quelconque des revendications précédentes, ledit module d'élasticité de la structure en treillis (11, 43, 71) adjacente à la surface de mise en prise (74) étant inférieur au module d'élasticité d'une partie proximale de la structure en treillis (11, 43, 71) espacée de la surface de mise en prise (74).

5. Implant orthopédique (1, 4, 7) selon l'une quelconque des revendications précédentes, ladite structure en treillis (11, 43, 71) comprenant une partie centrale (115b à c, 443a à c) et une partie périphérique (115a, 115d à h, 444a à c), ledit module d'élasticité de la partie périphérique (115a, 115d à h, 444a à c) étant inférieur au module d'élasticité de la partie centrale (115b à c, 443a à c).

6. Implant orthopédique (1, 4, 7) selon l'une quelconque des revendications précédentes, ledit module d'élasticité de l'élément de fixation (13) étant supérieur au module d'élasticité de la structure en treillis (11, 43, 71).

7. Implant orthopédique (1, 4, 7) selon l'une quelconque des revendications précédentes, ladite structure en treillis (11, 43, 71) comportant un module d'élasticité compris entre 3 GPa et 20 GPa.

8. Implant orthopédique (1, 4, 7) selon l'une quelconque des revendications précédentes, ledit élément de joint articulé étant un élément de joint articulé polymère (16, 73).

9. Implant orthopédique (1, 4, 7) selon l'une quelconque des revendications précédentes, ledit implant orthopédique (1, 4, 7) étant l'un quelconque parmi un implant de genou modulaire ou un implant de hanche modulaire et/ou ledit implant orthopédique (1, 4, 7) étant conçu comme l'un quelconque parmi un élément tibial, un élément fémoral ou une cupule acétabulaire.

10. Implant orthopédique (1, 4, 7) selon l'une quelconque des revendications précédentes, ladite structure en treillis (11, 43, 71) comprenant une pluralité d'entretoises reliées entre elles, et chaque entretoise comportant une dimension en coupe transversale comprise entre 0,1 mm et 0,5 mm ; et/ou ladite structure en treillis (11, 43, 71) comportant une densité d'entretoises comprise entre 3,0 par mm³ et 5,0 par mm³.

11. Implant orthopédique (1, 4, 7) selon l'une quelconque des revendications précédentes, ladite structure en treillis (11, 43, 71) comprenant une pluralité de nœuds, chaque nœud étant relié à au moins un autre nœud par une entretoise respective, ledit nombre moyen de raccordements entre la pluralité de nœuds étant compris entre 4,5 et 6,0.

12. Implant orthopédique (1, 4, 7) selon l'une quelconque des revendications précédentes, ladite structure en treillis (11, 43, 71) comportant un module d'élasticité anisotrope.

13. Implant orthopédique (1, 4, 7) selon l'une quelconque des revendications précédentes, ladite structure en treillis (11, 43, 71) représentant jusqu'à au moins 5 % de l'implant en volume.

14. Implant orthopédique (1) selon l'une quelconque des revendications précédentes, ladite structure en treillis (11) comprenant une pluralité de zones de renforcement (317a à d) comportant un module d'élasticité supérieur au premier module d'élasticité et au second module d'élasticité.
